(19) Europäisches Patentamt / European Patent Office / Office européen des brevets

(11) **EP 2 578 149 B1**

(12) **EUROPEAN PATENT SPECIFICATION**

(45) Date of publication and mention
of the grant of the patent:
**07.12.2016 Bulletin 2016/49**

(21) Application number: **11786814.1**

(22) Date of filing: **29.03.2011**

(51) Int Cl.:
*A61B 6/00* (2006.01)          *A61B 6/03* (2006.01)
*A61B 5/055* (2006.01)        *G01T 1/161* (2006.01)
*G01T 1/29* (2006.01)          *G01R 33/48* (2006.01)

(86) International application number:
**PCT/KR2011/002149**

(87) International publication number:
**WO 2011/149181 (01.12.2011 Gazette 2011/48)**

(54) **VARIABLE PET DEVICE**

VARIABLE PET-VORRICHTUNG

DISPOSITIF DE TOMOGRAPHIE PAR ÉMISSION DE POSITRONS VARIABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.05.2010 KR 20100050533**

(43) Date of publication of application:
**10.04.2013 Bulletin 2013/15**

(73) Proprietor: **Gachon University of
Industry-Academic
Cooperation Foundation
Gyeonggi-do 461-701 (KR)**

(72) Inventors:
• **CHO, ZangHee
Incheon 402-702 (KR)**
• **KIM, Young-Bo
Seongnam-si
Gyeonggi-do 463-756 (KR)**
• **SON, Young-Don
Bucheon-si
Gyeonggi-do 420-755 (KR)**
• **KIM, Hang-Keun
Incheon 405-717 (KR)**

(74) Representative: **Meyer-Dulheuer, Karl-Hermann
Dr. Meyer- Dulheuer & Partners LLP
Franklinstrasse 46-48
60486 Frankfurt am Main (DE)**

(56) References cited:
WO-A2-2009/125309          JP-A- 2004 049 924
KR-A- 20080 105 443          KR-A- 20100 122 596
US-A1- 2002 148 970

## Description

Technical Field

The present invention relates to a Positron Emission Tomography (hereafter, referred to as "PET") device, and particularly to a PET device that can change the diameter of a detection means surrounding a subject, according to the size of the subject.

Background Art

A Positron Emission Tomography (PET) is one of the nuclear medical imaging methods that can three-dimensionally display physiological, chemical and functional images of a human by using radiopharmaceuticals that emit positrons. At present, it is primarily used in diagnosing various cancers and is known as a method useful for differential diagnosis, staging, recurrence assessment, determination of curative effect, etc. for a cancer. In addition, receptor or metabolic images for assessing cardiac diseases, cerebral diseases and cerebral functions can also be obtained by means of PET.

A positron refers to an antiparticle that has a similar physical property to an electron having a negative (-) charge, but has a diametrically positive (+) charge. Such positrons are emitted from radioisotopes such as C-11, N-13, O-15, F-18, etc., as one kind of radiation, and since such elements are major constitutional elements of biomaterial, a radiopharmaceutical thereof can be utilized as a tracer for tracking a specific physiological, chemical or functional change. For example, since F-18-FDG, the radioactive medicine most frequently used, is a glucose-like material, it largely gathers in the area accelerated by a glucose metabolism, such as a cancer in a body, when it is administered.

A positron emitted from the radioisotope consumes all of its kinetic energy in a short time after emission and then undergoes annihilation by colliding with neighboring electrons, and at this time, it emits two (2) annihilation radiations (511 keV annihilation positrons; e.g., gamma-ray) at an angle of 180°. PET scanners that are cylindrically made are devices which can detect two (2) annihilation radiations emitted simultaneously. Upon reconstructing images by using the radiation detected thereby, it can then be displayed by way of a three-dimensional tomography image used for determining how many radiopharmaceuticals are gathered on any particular area of the body. The portion of the body displaying an abnormally strong signal due to an accumulation of radiopharmaceuticals in PET images can subsequently be diagnosed as a cancer.

However, while a PET scanner can provide molecular and functional information for human tissue, since it essentially has low resolution, it is limited in providing anatomical information. Therefore, recently, in order to overcome such issues, Positron Emission Tomography-Computer Tomography (PET-CT) scanners, Positron Emission Tomography-Computer Tomography-Magnetic Resonance Imaging (PET-MRI) scanners, etc., which are combined with Computer Tomography (CT) scanners or Magnetic Resonance Imaging (MRI) scanners, have become widely used. In the present specification, PET scanners, PET-CT scanners, PET-MRI scanners, etc. are all commonly referred to as "PET device".

Disclosure

Technical Problem

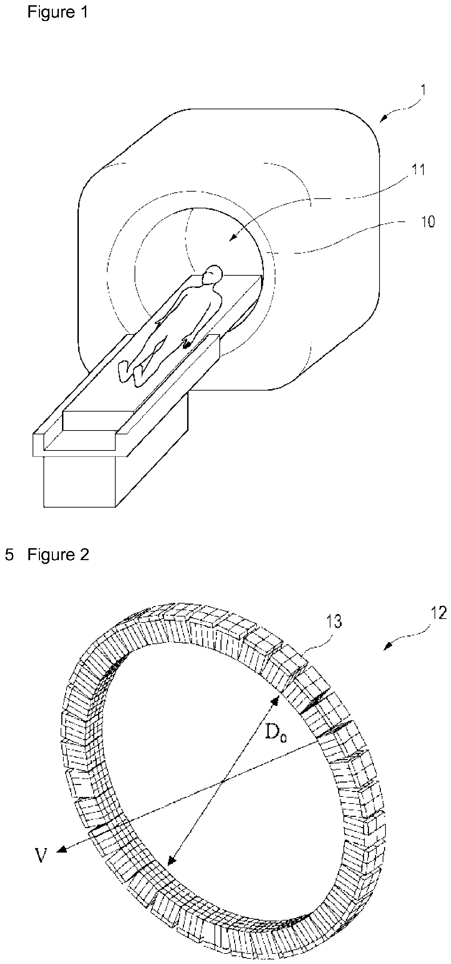

In a PET device, sensitivity and resolution are very important elements in assessing its performance. PET devices primarily used for human beings at present can be divided into PET devices only for brains and PET devices for the whole body (1) as shown in Fig. 1, corresponding to the subject being filmed. If it is assumed that they have the same length in the longitudinal axis (V) plane, a PET device for the whole body (1) in which the diameter $(D_o)$ of detection means (12) is large and therefore has a sensitivity much lower than that of a PET device utilized only for mapping the brain (1), in which the diameter of the detection means is small. This is due to the geometrical Solid Angle Coverage Ratio of PET devices for the whole body (1) being smaller than the Solid Angle Coverage Ratio of PET devices used only for the brain. However, since PET devices used only for the brain have a small diameter of the detection means, it is not viable to photograph a large subject such as the body of a patient. Therefore, it can be said that the optimal applicability of the two devices is wherein brain PET devices are used when photographing small subjects such as the brain, and whole body PET devices are used when photographing subjects of a larger size.

However, since PET devices are very expensive equipment, it can be financially uneconomical for an owner to simultaneously maintain both a PET device only for the brain and a PET device for the whole body. For the above reason, most medical institutions such as hospitals primarily use PET devices for a whole body to simultaneously cover various areas of the body. PET devices only for the brain are utilized only in research institutes within pharmaceutical, neuroscience and the like fields that require high-performance PET devices.

Alternatively, in WO2009/125309 and in US2002/0148970 PET devices with variable geometries are disclosed.

Technical Solution

An object of the present invention is to provide a PET device which can be used regardless of the size of the subject and can also provide high sensitivity when photographing small subjects such as a brain.

A PET device according to the present invention comprises a body having an opening therein to receive the subject and a detection means positioned to surround said subject in the circumferential direction of the opening. The detection means detects the radiation emitted by the subject. Additionally, the PET device comprises driving means driving the detection means. The detection means comprises detection heads of the first group arranged in a circumferential direction along the opening and, detection heads of the second group each disposed between the detection heads of the first group. The detection heads of the first group are moveable in a radial direction along the detection means by way of the driving means.

The PET device being further defined by the features of the independent claim and by optional features according to the dependent claims.

Advantageous Effects

According to the present invention, the diameter of the detection means detecting the radiation emitted by the subject can be expanded or contracted, such that the present invention can be used regardless of the size of the subject, and in particular, it can provide high sensitivity when small subjects such as the brain are filmed. Therefore, a user does not need to maintain both a PET device only for the brain and a PET device for the whole body, thereby easing the financial burden.

In addition, the detection means of the present invention is comprised of the detection heads of the first group arranged in a circumferential direction, and the detection heads of the second group disposed between the detection heads of the first group. The detection heads of the first group are moveable along the inner radial direction until the extreme end of each detection head reaches to the extreme end of the adjacent detection heads opposite each other, and additionally the detection heads of the second group are moveable along the inner radial direction until the upper end thereof reach to the detection heads of the first group. Therefore, according to the present invention, since the detection heads of the second group disposed behind the detection heads of the first group can detect photons passing through gaps between the detection heads of the first group, a reduction of sensitivity due to the presence of gaps can be avoided. Meanwhile, according to the present invention, even though photons are emitted to the detection heads of the first group at a high angle of incidence, there are many cases wherein they are emitted to the detection heads of the second group located behind them at a relatively low angle of incidence. Therefore, errors due to the degree of the angle of incidence corresponding to the radiation, which reduces resolution, can be decreased compared with the case where only the detection heads of the first group are used. Simultaneously with this, an improvement in the sensitivity can be obtained along with an increase of photons having small angle of incidence, which has a high efficiency of detection.

Description of Drawings

Fig. 1 is a perspective drawing of a PET device according to the prior art.

Fig. 2 is a perspective drawing of a detection means of PET device according to the prior art.

Fig. 3 depicts a figure in which the detection heads of the first group of the detection means and, the detection heads of the second group of the detection means are fixed, in the mode that the PET device according to the present invention photographs the body of the subject.

Fig. 4 depicts the figure that the detection heads of the first group of the detection means move along the inner radial direction such that the extreme end of each detection head of the first group reaches to the extreme end of the adjacent detection heads opposite each other, in the mode that the PET device according to the present invention photographs the brain of the subject.

Fig. 5 depicts the figure that the upper side of the detection heads of the second group reach to the detection heads of the first group, in the state that the extreme end of each detection head of the first group reaches to the extreme end of the adjacent detection heads opposite each other, in the mode that the PET device of the present invention photographs the brain of the subject.

Fig. 6 depicts the figure that the upper side of detection heads of the second group surrounds the detection heads of the first group at an interval corresponding to the detection heads of the first group, at the state before that where the extreme end of each detection head of the first group reaches to the extreme end of the adjacent detection heads opposite each other in the PET device according to the present invention.

Fig. 7 schematically depicts a side view of the detection means of the PET device according to the present invention.

Fig. 8 compares the minimum diameter of the detection means in the mode that the PET device of the present invention photographs the body with that in the mode that it photographs the brain.

Fig. 9 depicts the Solid Angle Coverage Ratio (Dead Space is not considered) according to a change of the minimum diameter of the detection means.

Fig. 10 schematically depicts the figure that radiation photons emitted from a patient are passed through the detection heads or are absorbed by the detection heads, in the state that the detection heads of the first group and the detection heads of the second group of the PET device according to the present invention are moved along the inner radial direction.

Mode for Invention

Hereinafter, the present invention is explained in detail in reference to the drawings attached to the present specification.

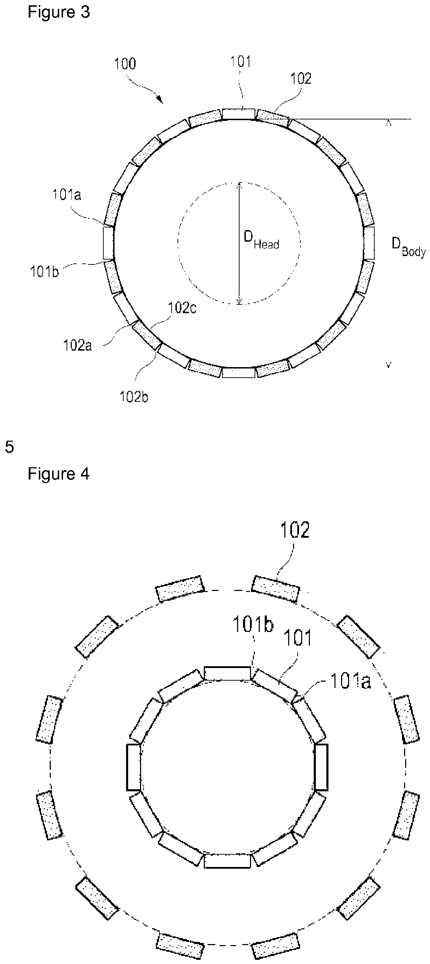

Figure 3 depicts the figure that the detection heads (101) of the first group and the detection heads (102) of the second group of the detection means (100) are fixed, in the mode (first mode) that the PET device according to the present inventions films the body of the subject. The PET device (1) includes the body (10) that an opening (11) for receiving the subject (patient) is formed therein and the detection means (100) that the opening (11) is located at so as to surround the subject in a circumferential direction relative to the opening (11). The detection means (100) can be a ring-shape or polygon-shape. The detection device (100) can detect the radiation emitted from the subject. The PET device (1) can contain driving means (not shown) driving the detection means (100). In one example of the present invention, the driving means can connect the body (10) and the detection means (100), and can be located at the body (10). The detection means (100) can contain the detection heads (101) of the first group being arranged in the circumferential direction to the opening (11), and the detection heads (102) of the second group each disposed between the detection heads (101) of the first group.

According to one example of the present invention, one extreme end (101a) of the detection heads (101) of the first group in the first mode can be fixed in a state facing the opposite extreme end (102b) of the detection heads (102) of the second group. Other side end (101b) of the detection heads (101) of the first group can be fixed in a state facing the opposite extreme end (102a) of the detection heads (102) of the second group. Therefore, the number of the radiation photons passing between gaps of the detection heads (101) of the first group and the detection heads (102) of the second group can be minimized. This can consequently increase the sensitivity of PET device in the first mode.

According to one example of the present invention, both the diameter ($D_1$) of the detection heads (101) of the first group and, the diameter ($D_2$) of the detection heads (102) of the second group in the first mode can be substantially the same with the first mode diameter ($D_{Body}$). Wherein, the numerical value of the first mode diameter ($D_{Body}$) can be appropriately selected from the numerical values of the diameter of the detection means of a conventional PET device for the whole body. In general, the numerical value of the diameter of the detection means of a PET device for the whole body has the range of 80 cm - 100 cm.

Meanwhile, according to one example of the present invention, a PET device can be converted from the first mode photographing the body of the subject to the second mode photographing the brain of the subject. Upon converting into the second mode, the detection heads (101) of the first group can be moved along the radial direction, in particular, to the inner radial direction by way of the driving means. The driving means, for example, can be consisted of a linear motor, ball screw, etc., and any one of them can be used if it is a means that can move the detection heads linearly. In the second mode, as shown in Figure 4, the extreme end (101 a, 101 b) of each detection heads (101) of the first group can be facing the opposite extreme end (101 a, 101 b) of the adjacent detection heads (100), respectively. According to one example of the present invention, the detection heads (101) of the first group can be moved along a radial direction of the detection means (100) with an identical distance. The diameter ($D_1$) that the detection heads (101) of the first group form can be substantially the same with the second mode diameter ($D_{Head}$). Also, the second mode diameter ($D_{Head}$) can be appropriately selected from the numerical values of the diameters of the detection means of the conventional PET device only for the brain.

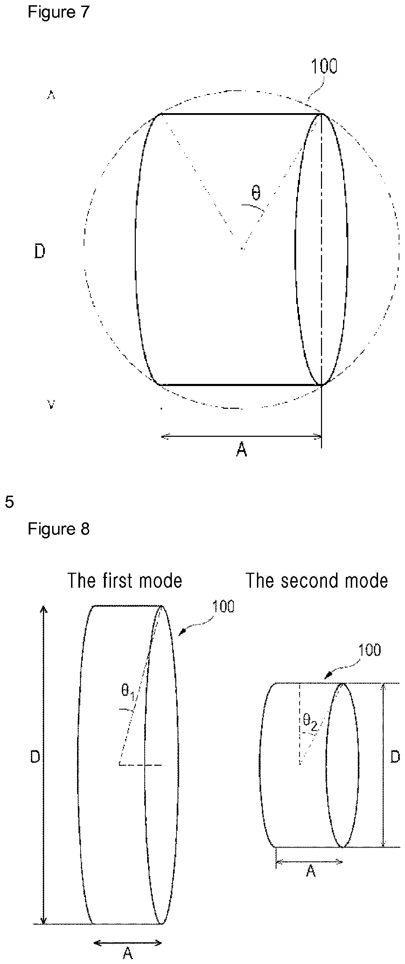

Figure 7 schematically depicts the figure when the detection means (100) of a PET device according to one embodiment of the present invention is viewed from the side. Figure 8 compares the minimum diameter of the detection means (100) in the first mode (D; that is, the diameter ($D_1$, $D_2$) of the detection heads (101, 102) of the first and second groups) and, the minimum diameter of the detection means (100) in the second mode (D; that is, the diameter ($D_1$) of the detection heads (101) of the first group) with each other. The minimum diameter (D) of the detection means (100) in the second mode is larger than the minimum diameter (D) of the detection means (100) in the first mode as shown in Figure 8. On the other hand, the longitudinal lengths (A) of the detection means in the first and second modes are substantially the same with each other. Solid Angle Coverage Ratio has a value of $\dfrac{4\pi * \sin\theta}{4\pi} * 100(\%)$ in case of the detection means (100) of a PET device as shown in Fig. 7. Therefore, the Solid Angle Coverage Ratio in the second mode has a value higher than the Solid Angle Coverage Ratio in the first mode.

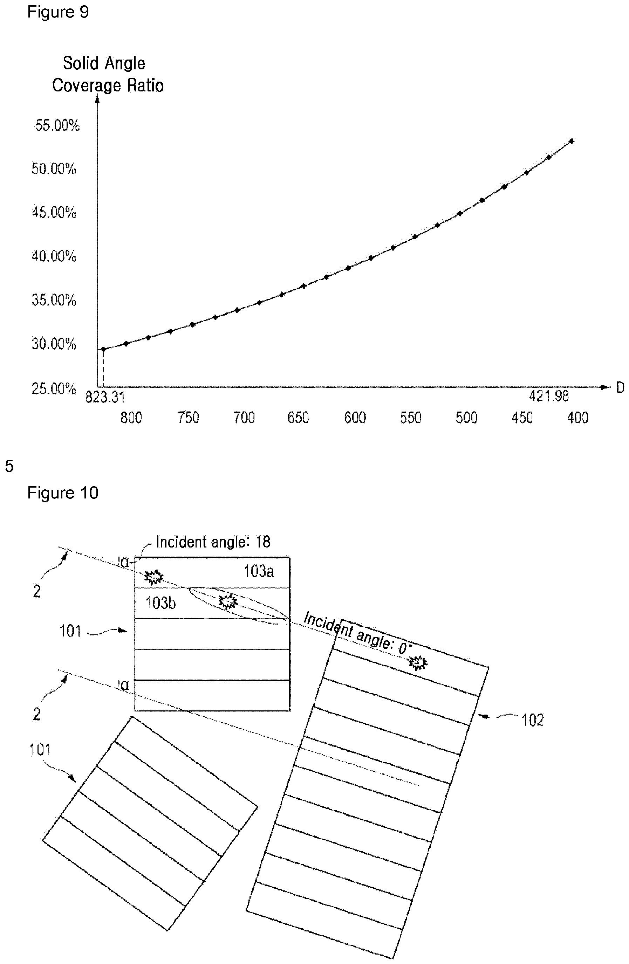

For example, when the minimum diameter (D) of the detection means (100) in the first mode is 823.31 mm, the minimum diameter (D) of the detection means (100) in the second mode is 421.98 mm, and the longitudinal length (A) of the detection means (100) is 253.4 mm, it is represented that the Solid Angle Coverage Ratio in the first mode is 29%, while the Solid Angle Coverage Ratio in the second mode is 52%, as shown in the experimental table of Fig. 9. For reference, Dead Space that cannot actually detect the radiation due to gaps between the detection heads, etc. was not considered in the experiment of Fig. 9. The Solid Angle Coverage Ratio refers to the geometrical ratio corresponding to the amount of radiation emitted from the patient can that reach the detection means, and in the case that the Solid Angle Coverage Ratio is high, this means that the sensitivity of the detection means is high to an equivalent extent. That is, since the sensitivity in the second mode is higher than that in the first mode when considering only the Solid Angle Coverage Ratio, a PET device according to the present invention can provide a PET image having sensitivity higher than that conventionally obtainable in the photographing of a small subject such as the brain. That is, the PET device of the present invention can provide a PET image having the best sensitivity that the relevant subject could optimally have, regardless of the size of the subject, by altering the diameter of the detection means detecting the radiation emitted by the subject. Also, with respect to the user, there is a financial gain in that there is no need to maintain both a PET device only for the brain and a PET device for the whole body.

When the PET device is converted from the first mode to the second mode, the detection heads (102) of the second group can also be moved along the inner radial direction by way of the driving means, in a method identical to that of the detection heads (101) of the first group. The detection heads (102) of the second group can also be moved along the radial direction of the detection means with an identical distance. Figure 10 depicts the figure wherein the radiation photons (2) omitted from the patient are passed through the detection heads (101, 102) or absorbed by the detection heads, in the state that all of the detection heads (101) of the first group and the detection heads (102) of the second group of a PET device according to the present invention are moved along the inner radial direction.

As shown in Figure 10, the radiation photons (2) emitted by the patient are firstly absorbed into the detection heads (101) of the first group, or passed through them. The photons (2) emitted around the gap between the detection heads (101) of the first group among the radiation photons (2) cannot be sufficiently absorbed by the detection heads (101), and most of them can be emitted outside. That is, since the photons emitted into the gap between the detection heads (101) of the first group are rarely detected, said gap part can be regarded as Dead Space. This Dead Space is present in all of the conventional PET devices consisting of the fixed detection heads (13) as shown in Fig. 2. In the present invention, in order to solve the problem that the Solid Angle Coverage Ratio is lowered due to said Dead Space, the detection heads (102) of the second group are also allowed to move along the inner radial direction, together with the detection heads (101) of the first group. As shown in Fig. 10, the detection heads (102) of the second group located behind the detection heads (101) of the first group can as a backup mechanism absorb the photons (2) that passed by the gap between the detection heads (101) of the first group. As a result, the phenomenon wherein sensitivity is lowered due to the presence of the gap can be avoided.

Meanwhile, the minimum diameter of the detection means (100) in the second mode is smaller than that of the detection means (100) in the first mode. Therefore, the angle of incidence ($\alpha$) that the photons emitting from the outside part (i.e., outside part of FOV (Field of View) to the longitudinal axis (V)) of the patient in the second mode emit into the detection heads (101) of the first group, is larger than the angle of incidence in the first mode. If the angle of incidence ($\alpha$) of the radiation photons is large, the amount of the photons (2) emitted outside by passing through the detection head (101) of the first group is increased and the resolution of the PET device can also be lowered to an equivalent extent. Figure 10 depicts the figure that the photons (2) are emitted to the detection element (103a) of the detection heads (101) of the first group at an angle of incidence of 18°. Similar to the above, there are many cases where the photons injected at a large angle of incidence are detected at the detection element (103b) disposed behind the detection element (103a) being firstly emitted to, and not at the firstly emitted to detection element (103a). This can lower the space resolution. However, according to one embodiment of the present invention, the detection heads (102) of the second group can be disposed behind the detection heads (101) of the first group. In this case, even though photons (2) are emitted to the detection heads (101) of the first group at a the large angle of incidence, they can be emitted to the detection heads (102) of the second group disposed behind direction at a relatively small angle of incidence (0° in Fig. 10). Therefore, the detection heads (102) of the second group can detect photons passed through the detection heads (101) of the first group with lager number of exact LORs (Line of Response). That is, even though many LORs are not precisely detected by the detection heads (101) of the first group, many of said LORs can be precisely detected by the detection heads (102) of the second group. With this, a PET device according to the present invention can reduce errors due to the angle of incidence of radiation, compared to the case using only the detection heads of the first group, and consequently can increase the resolution of a PET device. Simultaneously with this, as photons having a small angle of incidence having high detection efficiency are increased, sensitivity can be improved thereof.

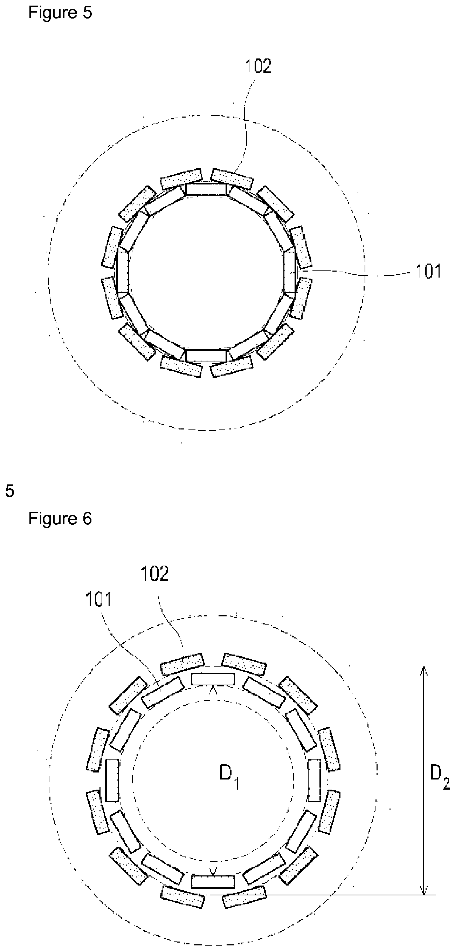

According to one embodiment of the present invention, a PET device can be converted between the first, second and third modes. The third mode is an intermediary mode of the brain-filming mode and body-filming mode, and is used to obtain an optimal sensitivity and resolution with a subject being larger than the brain and smaller than the body. In the third mode, the detection heads (101) of the first group can be moved along the inner radial direction until the extreme ends (101 a, 101 b) of each detection head (101) of the first group reach to the extreme ends (101 a, 101b) of adjacent detection heads (101) opposite each other as shown in Fig. 6. The diameter ($D_1$) of the detection heads (101) of the first group at this time can be appropriately selected depending on the section diameter of the subject. According to one embodiment of the present invention, the detection heads (102) of the second group can also be moved along the inner radial direction by way of the driving means. The detection heads (102) of the second group can be moved so as to dispose behind the detection heads (101) of the first group, and can be moved until the upper side (102c) thereof reaches to the detection heads (101) of the first group. Also, the detection heads (102) of the second group can be moved along the inner radial direction such that the upper side (102c) thereof surrounds the detection heads (101) of the first group at a set interval with the detection heads (101) of the first group.

## Claims

1.  A Positron Emission Tomography (PET) device including detection means (100) located so as to surround a subject to detect radiation emitted by said subject; and driving means driving said detection means (100), wherein said detection means (100) includes detection heads of a first group (101), arranged in the circumferential direction thereof and detection heads of a second group (102) each disposed between said detection heads of the first group (101), said detection heads of the first group (101) can be moved along the radial direction of said detection means (100) by way of said driving means, said detection heads of the second group (102) can be moved along the radial direction of said detection means (100) by way of said driving means, said detection heads of the second group (102) move together with said detection heads of the first group (101) to increase the solid angle coverage ratio of said detection means (100), an operation mode of the PET device is convertible from a first mode to a second mode, the detection means (100) having first diameter ($D_{Body}$) in the first mode and having second diameter ($D_{Head}$) in the second mode, when the operation mode of the PET device is converted from the first mode to the second mode, the detection heads of the first group (101) and the detection heads of the second group (102) move linearly along the inner radial direction of said detection means (100), in the first mode, the detection heads of the first group (101) and the detection heads of the second group (102) are disposed in a circumference of the first diameter ($D_{Body}$), and the detection heads of the second group (102) are disposed respectively between the detection heads of the first group (101), when converting in the second mode, the detection heads of the first group (101) are moved to the inside of the radial direction such that they are disposed in a circumference of the second diameter ($D_{Head}$) for firstly absorbing the radiation photons emitted from the subject, and the detection heads of the second group (102) are moved with an identical distance to the inside of the radial direction until the upper sides thereof reach the detection heads of the first group (101) or until the upper sides thereof surround the detection heads of the first group (101) at a set interval, such that the detection heads of the second group (102) are disposed to surround the detection heads of the first group (101) and to cover the gaps between the detection heads of the first group (101) for secondly absorbing the radiation photons passing through the gaps.

2.  The PET device according to claim 1, wherein said detection heads of the first group (101) are moveable with an identical distance along the radial direction of said detection means (100).

3.  The PET device according to claim 1, wherein said detection heads of the first group (101) are moved along the inner radial direction until the extreme end of each detection head reaches the extreme end of the adjacent detection heads opposite each other

4.  The PET device according to claim 3, wherein said detection heads of the first group (101) are moved along the inner radial direction until just prior to the state wherein the extreme end of each detection head reaches the extreme end of the adjacent detection heads opposite each other.

5.  The PET device according to claim 1, wherein said detection (100) is a ring shape or polygon shape.

6.  The PET device according to claim 1, wherein said driving means is a linear motor or ball screw.

7.  The PET device according to claim 1, further comprising a body having an opening therein to receive said subject; and wherein said detection means (100) is located so as to surround said received subject in the circumferential direction relative to said opening to thereby detect radiation emitted by said subject.

## Patentansprüche

1.  Eine Positronen-Emissions-Tomographie (PET) Vorrichtung mit Detektionsmittel (100), die derart angeordnet sind, dass diese einen Gegenstand umgeben, um die durch den Gegenstand emittierte Strahlung zu detektieren; und Antriebsmittel, die das Detektionsmittel (100) antreiben,

wobei das Detektionsmittel (100) eine erste Gruppe Detektionsköpfe (101) in Umfangsrichtung und eine zweite Gruppe Detektionsköpfe (102) enthält, die jeweils zwischen den Detektionsköpfen der ersten Gruppe (101) angeordnet sind,

die erste Gruppe Detektionsköpfe (101) mittels der Antriebseinrichtung entlang der radialen Richtung der Detektionsmittel (100) bewegt werden kann,

die zweite Gruppe Detektionsköpfe (102) mittels der Antriebseinrichtung entlang der radialen Richtung der Detektionsmittel (100) bewegt werden kann,

sich die zweite Gruppe Detektionsköpfe (102) mit der ersten Gruppe Detektionsköpfen (101) zusammen bewegt, um den Deckungsgrad der Raumwinkel der Detektionsmittel (100) zu erhöhen,

ein Betriebsmodus der PET-Vorrichtung von einem ersten Modus in einen zweiten Modus umwandelbar ist, wobei die Detektionsmittel (100) einen ersten Durchmesser ($D_{Körper}$) im ersten Modus und einen zweiten Durchmesser ($D_{Kopf}$) im zweiten Modus aufweisen,

wenn der Betriebsmodus der PET-Vorrichtung von dem ersten Modus in den zweiten Modus umgewandelt wird, sich die erste Gruppe Detektionsköpfe (101) und die zweite Gruppe Detektionsköpfe (102) linear entlang der inneren radialen Richtung der Detektionsmittel (100) bewegen,

in dem ersten Modus, die erste Gruppe Detektionsköpfe (101) und die zweite Gruppe Detektionsköpfe (102) in einem Umfang des ersten Durchmessers ($D_{Körper}$) angeordnet sind und die zweite Gruppe Detektionsköpfe (102) jeweils zwischen den Detektionsköpfen der ersten Gruppe (101) angeordnet sind, bei der Umwandlung in den zweiten Modus die erste Gruppe Detektionsköpfe (101) an der Innenseite der radialen Richtung bewegt werden, so dass sie in einem Umfang des zweiten Durchmessers ($D_{Kopf}$) angeordnet sind, um zuerst die von dem Gegenstand emittierte Strahlungsphotonen zu absorbieren,

und die zweite Gruppe Detektionsköpfe (102) mit einem gleichen Abstand zu der Innenseite der radialen Richtung bewegt wird, bis die Oberseiten der Detektionsköpfe die erste Gruppe Detektionsköpfe (101) erreichen oder bis die Oberseiten der Detektionsköpfe die erste Gruppe Detektionsköpfe (101) in einem festgelegten Intervall umgeben, so dass die zweite Gruppe Detektionsköpfe (102) derart angeordnet ist, dass sie die erste Gruppe Detektionsköpfe (101) umgibt und dass sie die Lücken zwischen den Detektionsköpfen der ersten Gruppe (101) abdeckt, um die weiteren Strahlungsphotonen zu absorbieren, die durch die Lücken dringen.

2. PET-Vorrichtung nach Anspruch 1, wobei die erste Gruppe Detektionsköpfe (101) mit einem gleichbleibenden Abstand entlang der radialen Richtung der Detektionsmittel (100) bewegbar ist.

3. PET-Vorrichtung nach Anspruch 1, wobei die erste Gruppe Detektionsköpfe (101) entlang der inneren radialen Richtung bewegt wird, bis das äußerste Ende jedes Erfassungskopfes das äußerste Ende der benachbarten gegenüberliegenden Detektionsköpfe erreicht.

4. PET-Vorrichtung nach Anspruch 3, wobei die erste Gruppe Detektionsköpfe (101) entlang der inneren radialen Richtung bewegt wird, bis kurz vor die Position, in der das äußerste Ende jedes Erfassungskopfes das äußerste Ende der benachbarten gegenüberliegenden Detektionsköpfe erreicht.

5. PET-Vorrichtung nach Anspruch 1, wobei die Detektionsmittel (100) in einer Ringform oder Polygonform angeordnet sind.

6. PET-Vorrichtung nach Anspruch 1, wobei das Antriebsmittel ein Linearmotor oder eine Kugelumlaufspindel ist.

7. PET Vorrichtung nach Anspruch 1, zusätzlich aufweisend einen Körper mit einer Öffnung um den Gegenstand aufzunehmen; und

wobei die Detektionsmittel (100) derart angeordnet sind, um den aufgenommen Gegenstand in Umfangsrichtung bezogen auf die Öffnung zu umgeben, um dadurch die vom Gegenstand emittierte zu erfassen.

**Revendications**

1. Un appareil tomoscintigraphie par émission de positons (TEP) comportant des moyens de détection (100), qui sont disposés de manière à entourer un objet pour détecter le rayonnement émis par l'objet; et des moyens d'entraînement qui entraînent les moyens de détection (100),

dans lequel lesdits moyens de détection (100) comprend un premier groupe de têtes de détection (101) dans la direction circonférentielle et un second groupe de têtes de détection (102), chacun étant disposé entre les têtes du premier groupe de détection (101),

le premier groupe de têtes de détection (101) peut être déplacé par le moyen d'entraînement le long de la direction radiale des moyens de détection (100),

le second groupe de têtes de détection (102) peut être déplacé par le moyen d'entraînement le long de la direction radiale des moyens de détection (100),

le second groupe de têtes de détection (102) est déplacées ensemble avec le premier groupe de têtes de détection (101) afin d'augmenter le degré de couverture des angles solides des moyens de détection (100),

un mode de fonctionnement de l'appareil TEP est convertible d'un premier mode à un second mode, dans lequel les moyens de détection (100) comprennent un premier diamètre ($D_{Corps}$) dans le premier mode et un second diamètre ($D_{Tête}$) dans le second mode

lorsque le mode de fonctionnement de l'appareil TEP est converti du premier mode au second mode, le premier groupe de têtes de détection (101) et le second groupe de têtes de détection (102) se déplacent de façon linéaire le long de la direction radiale interne des moyens de détection (100),

dans le premier mode, le premier groupe de têtes de détection (101) et le second groupe de têtes de détection (102) sont disposées dans une circonférence du premier diamètre ($D_{corps}$) et les têtes de détection du second groupe (102) sont arrangés entre chacune des têtes du premier groupe de détection (101) respectivement,

pendant la conversion dans le second mode, le premier groupe de têtes de détection (101) est déplacé à l'intérieur de la direction radiale, de sorte qu'ils sont disposés dans une périphérie du second diamètre ($D_{Tête}$), pour pouvoir absorber le rayonnement des photons émis par l'objet,

et le second groupe de têtes de détection (102) est déplacé à une distance constante de l'intérieur de la direction radiale jusqu'à ce que les hauts des têtes de détection atteignent le premier groupe de têtes de détection (101) ou jusqu'à ce que les hauts des têtes de détection entourent les têtes du premier groupe de détection (101) dans un intervalle défini, de sorte que le second groupe de têtes de détection (102) est arrangé de telle sorte qu'il entoure le premier groupe de têtes de détection (101) et qu'il couvre les interstices entre les têtes du premier groupe de détection (101) pour absorber les autres photons du rayonnement qui pénètrent à travers les trous.

2. Appareil TEP selon la revendication 1, dans lequel le premier groupe de têtes de détection (101) est mobile à une distance défini le long de la direction radiale des moyens de détection (100).

3. Appareil TEP selon la revendication 1, dans lequel le premier groupe de têtes de détection (101) est déplacé le long de la direction radiale intérieure jusqu'à l'extrémité extérieure de chaque tête de détection atteint l'extrémité la plus externe de la tête de détection adjacente.

4. Appareil TEP selon la revendication 3, dans lequel le premier groupe de têtes de détection (101) est déplacé le long de la direction radiale intérieure, juste avant la position dans laquelle l'extrémité extérieure de chaque tête de détection atteint l'extrémité la plus externe de la tête de détection adjacente.

5. Appareil TEP selon la revendication 1, dans lequel les moyens de détection (100) sont disposées en une forme annulaire ou une forme de polygone.

6. Appareil TEP selon la revendication 1, dans lequel le moyen d'entraînement est un moteur linéaire ou une vis à billes.

7. Appareil TEP selon la revendication 1, comprenant en outre un corps avec une ouverture pour recevoir l'objet; et
dans lequel les moyens de détection (100) sont arrangés pour entourer l'objet dans la direction circonférentielle par rapport à l'ouverture pour détecter le rayonnement émis par l'objet.

Figure 1

Figure 2

Figure 3

Figure 4

Figure 5

Figure 6

Figure 7

Figure 8

The first mode    The second mode

Figure 9

Solid Angle
Coverage Ratio

Figure 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009125309 A
- US 20020148970 A